# EUROPEAN PATENT SPECIFICATION

(11) **EP 2 637 602 B1**
(45) Date of publication and mention of the grant of the patent: **04.11.2015**
(21) Application number: 11805111.9
(22) Date of filing: 08.11.2011
(51) Int. Cl.: A61F 2/06

(54) **CURVED STENT GRAFT ASSEMBLY**
GEKRÜMMTE STENTPROTHESENANORDNUNG
ENSEMBLE GREFFE D'ENDOPROTHÈSE INCURVÉE

(30) Priority: 08.11.2010 GB 201018869
(43) Date of publication of application: 18.09.2013
(73) Proprietor: Isis Innovation Limited, Summertown, Oxford OX2 7SG (GB)
(72) Inventor: ZHOU, Xiang, Oxford OX1 3PJ (GB); YOU, Zhong, Oxford OX1 3PJ (GB)
(74) Representative: Merryweather, Colin Henry
(86) International application number: PCT/GB2011/001569
(87) International publication number: WO 2012/063015

(56) References cited:
- EP-A1- 1 506 793
- WO-A1-03/034948
- WO-A2-2008/051543
- US-A1- 2009 099 650

## Description

The present invention relates to a stent graft assembly (often referred to just as a stent graft) that is curved at a treatment site.

A stent graft assembly is a medical device designed to be delivered to a lumen at a treatment site in the human (or even animal) body, for instance the aorta, a coronary artery, or the oesophagus etc. and comprises a graft of flexible material having the shape of a tube supported by a stent frame. Stent graft assemblies provide a medical treatment of some sort and are typically used in endovascular surgery, for example to treat an aneurysm such as an aortic aneurysm. An aortic aneurysm is a weak area that develops in the wall of the aorta, the major artery that carries blood from the heart, through the chest and abdomen, to the rest of the body. While the stretched vessel may occasionally cause discomfort, a greater concern is the risk of rupture, which causes severe pain; massive internal hemorrhage; and, without prompt treatment, results in a quick death. Aortic aneurysms are classified by where on the aorta they occur. Abdominal aortic aneurysms (AAA) are found on the abdominal aorta and are the most common form of aortic aneurysm. Thoracic aortic aneurysms (TAA) are found on the thoracic aorta. Aortic aneurysms may be treated by a stent graft assembly in technique known as Endovascular Aneurysm (or Aortic) Repair (EVAR), as an alternative to more dangerous open surgery repair.

To facilitate delivery of the stent graft assembly to a treatment site in lumen, the stent graft assembly needs to be capable of being collapsed to reduce its outer dimensions. This is to facilitate the passage of the stent into the treatment site in the lumen where the stent is expanded for deployment. To achieve this, the stent frame may be configured to be deformable between an expanded state and a radially collapsed state. For example, the stent frame may comprise a series of rings of wire, each extending around the longitudinal axis of the graft in a zig-zag shape that allows the rings of wire to collapse and expand by closing and opening of the zig-zag shape. The graft of flexible material collapses and expands with the stent frame, either elastically or by folding or a combination thereof.

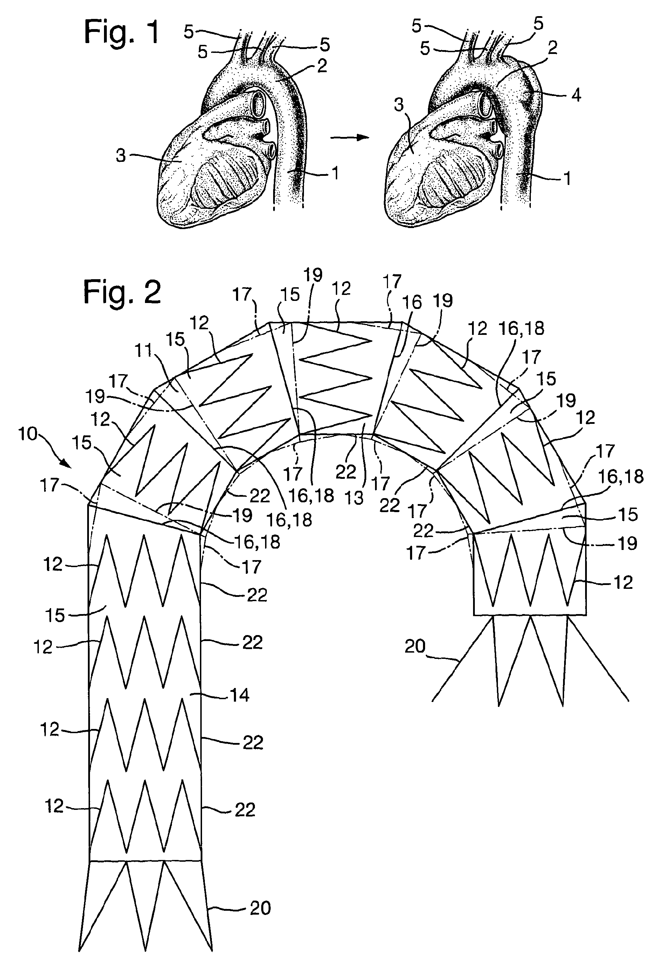

In some types of treatment, the stent graft assembly needs to be deployed at a treatment site in a lumen that is curved. An example of this is treatment of a thoracic aortic aneurysm (TAA). As shown in Fig. 1, the thoracic aorta 1 has an arch 2 that is curved close to where the aorta 1 is connected to the heart 3, and an aneurysm 4 may form in the region of the arch 2. The TAA may be treated by deploying a stent graft assembly to the site of the aneurysm 4 inside the arch 2 of the thoracic aorta 1. It is noted that further arteries 5 branch from the arch 2 of the aorta 1 adjacent the aneurysm 4.

For such deployment at a treatment site in a lumen that is curved, known stent graft assemblies are typically manufactured in a straight configuration but provided with sufficient flexibility to be delivered to the treatment site and deformed into an expanded state in which the stent graft assembly conforms to the curved shape of the lumen. It is straightforward to provide the stent frame with sufficient flexibility to achieve this. For example, where the stent frame comprises a series of rings of wire, each extending around the longitudinal axis of the graft in a zig-zag shape, the rings of wire may change orientation relative to each other, for example by including flexible linkages, or more commonly no linkages, therebetween. As to the graft of flexible material, this is configured to deform from the shape of a tube that is curved along a longitudinal axis of the tube when the graft is in an expanded, undeformed state into the curved shape. This deformation is often achieved by making the graft sufficiently flexible to accommodate this curvature elastically or by folding inside the curve. Alternatively, this deformation may be achieved by providing the graft with annular, concertinaed folds that allow deformation of the graft to curve perpendicular to the longitudinal axis of the graft in any direction around a full 360°.

Whilst these arrangements provide the capability of deployment at a treatment site in a lumen that is curved, there are a number of technical difficulties. There can be physical and mechanical incompatibilities between the stent frame and the graft that lead to incomplete deployment of the stent graft assembly, uneven distribution of stresses, or even rupture and/or tearing of the graft during the expansion of the stent graft assembly. In the case of achieving the curved deformation by making use of the flexibility of the graft, this can in particular result in elastic stresses on the outside and/or inside of the curved shape and/or creases on the inside of the curved shape in the deployed state. In the case of achieving the curved deformation by providing the graft with annular, concertinaed folds, the degree of elastic stress is reduced but there are folds of greater extent in the deployed state. Furthermore, there is variation between patients in the shape and degree of curvature of the curved lumen that makes it difficult to design stent graft assemblies to fit different patients.

WO-2008/051543 discloses a stent member attached to a tubular graft. The graft is curved in its expanded, undeformed state, and buckles when deformed into a straight state.

The present invention is intended to alleviate some of these technical difficulties.

According to the present invention, there is provided a stent graft assembly comprising:
a graft of flexible material having the shape of a tube that is curved along a longitudinal axis of the tube when the graft is in an expanded, undeformed state; and
a stent frame that supports the graft and is configured to be deformable between an expanded state and a radially collapsed state, the stent frame comprises a series of rings, each extending around the longitudinal axis of the graft in a zig-zag shape,
wherein the stent graft assembly is deformable into a state in which the graft is deformed into the shape of a tube that is straight, and
characterized in that the stent graft assembly further comprises linkages arranged to maintain the separation between each adjacent pair of rings in said series, the linkages being arranged on the side of the graft that is inside the curved shape when the graft is in an expanded, undeformed shape.

Thus, the stent graft assembly in accordance with the invention differs from the known type of stent graft assembly described above in that the graft of flexible material has the shape of a tube that is curved along a longitudinal axis of the tube when the graft is in an expanded, undeformed state. In other words, the natural, undeformed state of the graft is curved, rather than the straight. This provides a number of benefits, in particular that when stent graft assembly is deployed at a treatment site in a curved lumen, the deformation of the graft is reduced, as compared to the graft having the shape of a tube that is straight when the graft is in an expanded, undeformed state. As a result of the reduced deformation when deployed, the various above-described difficulties in implementing the known type of stent graft assembly are reduced or avoided. Notably, the elastic stresses and/or creases in the graft when deployed in the curved lumen are reduced which assists deployment and reduces the risk of rupture and tearing.

Advantageously, the graft may be designed so that its shape when in an expanded, undeformed state conforms to the shape of the curved lumen so that there is no deformation of the graft when deployed. This may be achieved for example by scanning the lumen to determine its shape and manufacturing the graft to match the so determined shape.

As the graft has the shape of a tube that is curved when the graft is in an expanded, undeformed state, it is required to be deformed into a state in which the graft has the shape of a tube that is straight when the stent graft assembly as a whole is in a straight state, for example during delivery. This deformation of the graft to be straight may be achieved, for example, by folding of the graft or by elastic deformation of the graft, or by a combination thereof.

Advantageously, the stent graft assembly may be deformable into said state in which the graft is deformed into the shape of a tube that is straight by folding of the graft. Such folding provides the deformation mechanically, which can provide advantages. One advantage is that the folding makes the processes of deforming the graft prior to delivery and deploying the graft *in situ* easy to perform. This facilitates the overall process of delivering the stent graft assembly correctly. Another advantage is that the folding can reduce the generation of stresses within the material of the graft in the deformed state. This can reduce the risk of damage to the stent graft assembly during delivery.

As an example of folding having this advantage, the folding may occur along one or more pairs of annular fold lines that extend around the stent graft assembly, the fold lines in each pair diverging from each other from the inside of the curve of the tube to the outside of the curve of the tube. In this example, in respect of the or each pair, the graft folds in opposite senses on the fold lines of the pair causing the graft between the pair of fold lines to fold back over the graft. This folding thereby forms a flap of material that lies along the tubular shape of the graft.

The stent frame comprises a series of rings, each extending around the longitudinal axis of the graft in a zig-zag shape. The zig-zag shape allows radial collapse and expansion of the stent frame which facilitates delivery and deployment of the stent graft assembly. The stent graft assembly further comprises linkages arranged to maintain the separation between each adjacent pair of rings in the series, with the linkages arranged on the side of the graft that is inside the curved shape when the graft is in an expanded, undeformed shape. The linkages maintain the separation between rings and prevent them moving together causing deformation of the graft that is undesirable for the reasons discussed above and that would reduce the advantage of the invention. The linkages may also prevent relative rotation of the rings about the axis of the tubular shape during deployment that would introduce undesirable deformation of the graft. By arranging the linkages on the side of the graft that is inside the curved shape, which is the shortest side, the deformation in the deformed state in which the stent is in the shape of a straight tube involves a reduction in the length of the other sides of the graft. Such deformation is easily accommodated, for example by folding or creasing of the material of the graft. This limits stretching that could only be accommodated by elastic deformation to a degree that might introduce undesirable stresses in the material of the graft that risk causing damage.

The stent graft assembly may be manufactured simply by forming the graft of flexible material with the appropriate shape and supporting the graft on the stent frame. Advantageously, the graft may be supported on the stent frame when the stent frame is in said expanded state and when the graft is in said expanded, undeformed state, and so is curved. In this case, in order to put the stent graft assembly into a suitable state for delivery, the manufacturing method may further comprise deforming the stent graft assembly into a state in which the graft is deformed into the shape of a tube that is straight and then deforming the stent frame into said radially collapsed state, for delivery of the stent graft assembly. The stent graft assembly may be deployed by delivering the stent graft assembly to the treatment site in a curved lumen in this state and then deforming the stent frame into said expanded state.

The graft may be formed in a variety of ways.

In one type of embodiment, the graft may be moulded to have said shape of a tube that is curved when the graft is in an expanded, undeformed state. It may be moulded from any suitable material, for example a polymer

In another type of embodiment, the graft may be formed by pieces of flexible material joined together at seams in a configuration providing the graft with said shape of a tube that is curved when the graft is in an expanded, undeformed state.

To allow better understanding, embodiments of the present invention will now be described by way of non-limitative example with reference to the accompanying drawings, in which:
Fig. 1 is a pair of comparative drawings of a the aorta with and without a thoracic aortic aneurysm;
Fig. 2 is a side view of a curved stent graft assembly in a fully-expanded state;
Fig. 3 is a side view of a curved stent graft assembly of Fig. 2 deformed to straighten it for radial collapse;
Fig. 4 is a side view of the stent graft assembly shown in Fig. 3 after radial collapse;
Fig. 5 is a side view of the stent graft assembly shown in Fig. 4 delivered in a curved shape inside a catheter to the treatment site of a TAA in an aorta that is shown in cross-section;
Fig. 6 is a side view of the stent graft assembly shown in Fig. 5 after partially release from the catheter;
Fig. 7 is a side view of the stent graft assembly of Fig. 5 after complete release from the catheter and radial expansion; and
Fig. 8 is a flow chart of a method of manufacturing the curved stent graft assembly.

Figs. 2 to 4 show a stent graft assembly 10 designed for treatment of a TAA in which the lumen to be treated is the aorta 1 at a treatment site in the arch 2 where an aneurysm 4 has formed, as shown in Fig 1.

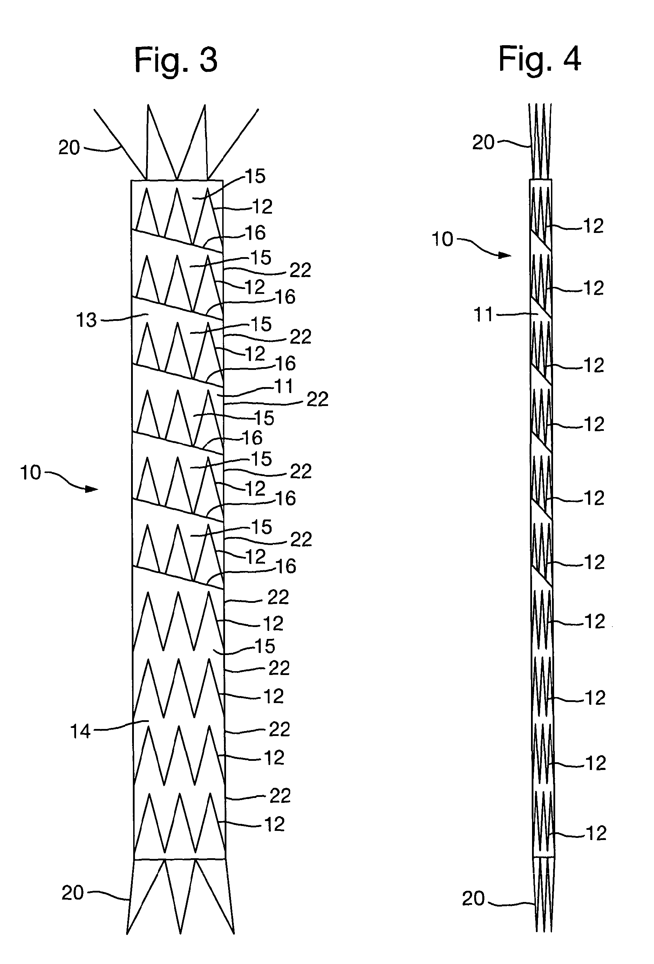

The stent graft assembly 10 comprises a graft 11 of flexible material supported on a stent frame formed by a series of rings 12 of wire, each ring 12 extending around the longitudinal axis of the graft 11 in a zig-zag shape. To provide radial collapse of the stent graft assembly 10 for delivery, the rings 12 are each deformable between an expanded state shown in Fig. 2 and a radially collapsed state shown in Fig. 4 by opening and closing of their zig-zag shape. This facilitates delivery and deployment of the stent graft assembly 10.

The rings 12 have a construction that is known in itself. The rings 12 may be manufactured using a conventional technique, such as by shaping a piece of wire or by cutting them from a tube of material, for example using laser cutting. The rings 12 may be made of any suitable biocompatible material, for example nitinol or stainless steel.

The rings 12 may have linkages 22 maintaining the separation between each adjacent pair of rings 12 in the series of rings 12 that are arranged as described below to provide flexibility along the longitudinal axis of the stent graft assembly 10.

The linkages 22 may each be separate elements arranged between the adjacent pairs of rings 12 in said series. A first example of this is that, if the rings 12 are manufactured by shaping a piece of wire, the linkages 22 may be portions of wire connected between the rings 12. In this case, the linkages 22 may be portions of the same piece of wire as used to form a ring 12, or separate pieces of wire. A second example of the linkages 22 being separate elements arranged between the adjacent pairs of rings 12 is that the linkages 22 and the rings 12 may all be integrally formed. For example, if the rings 12 are manufactured by cutting them from a tube of material, the linkages 22 may cut from the same tube of material.

Alternatively, the linkages 22 may be formed by a common element that is connected directly or indirectly to plural rings 12, for example a strut extending along the entire length of the stent graft assembly 10 or plural struts each extending along a part of the length of the stent graft assembly 10.

The linkages 22 have the advantage of assisting expansion of the graft from a collapsed straight shape to a curved expanded state, as described below.

The graft 11 is attached to the rings 12 by any suitable technique, for example by stitching or by bonding using a suitable biocompatible adhesive, or by the rings 12 being integrated into the graft 11, for example during the moulding process if the graft 11 is moulded.

The graft 11 of flexible material is shown in its expanded, undeformed state in Fig. 2, this being the natural state of the graft 11. In this state, the graft 11 has the shape of a tube that is curved along a longitudinal axis of the tube in first portion 13 and is straight in a second portion 14. The graft 11 is designed to conform with the shape of the aorta 1, in particular the first portion 13 that is curved being designed to conform with the shape of the arch 2 of the aorta 1 and the second portion 14 that is straight being designed to conform with the shape of the adjacent stretch of the aorta 1. A high degree of conformation may be achieved by scanning the patient using conventional imaging techniques to determine the precise shape of the aorta 1 and manufacturing the graft 11 to match the so determined shape. The linkages 22 are arranged on the side of the graft 11 on the inside of its curved shape.

The graft 11 may be formed with this shape in a variety of ways.

In a first type of embodiment of the graft 11 shown in Fig. 2 in bold outline, in which the graft 11 comprises pieces 15 of flexible material joined together at seams 16. The pieces 15 of flexible material may be joined together by any suitable technique, for example by stitching, by bonding using a suitable adhesive, for example a polymer, or by welding, for example heat welding or laser welding. The pieces 15 of flexible material are in the following configuration. Each individual piece 15 of flexible material has the shape of a tube section that is straight along a respective longitudinal axis of the tube section. The seams 16, however, are angled with respect to the respective longitudinal axis of the tube sections to provide the graft 11 with said shape of a tube that is curved in the first portion 13.

In a second type of embodiment of the graft 11, the graft 11 is moulded to have said shape of a tube that is curved in the first portion 13. In this second type of embodiment, the graft 11 has a smoothly curved profile in the first portion 13, shown by the dashed lines 17 in Fig. 2. In this case, the graft 11 may be formed from a single piece of material.

In addition, the stent graft assembly 10 has an anchor 20 at each end. The anchors 20 are attached to the graft 11 but protrude therefrom. Each anchor 20 is a ring extending around the longitudinal axis of the graft 11 in a zig-zag shape, in a similar manner to the rings 12, but being a conical section extending radially outwardly distal from the graft 11. The anchors 20 have the purpose of engaging the lumen when the stent graft assembly 10 is deployed. Alternatively the anchors 12 could be replaced by any other suitable form of anchor such as hooks.

The stent graft assembly 10 may be manufactured in the state shown in Fig. 2 using the techniques described in more detail below. Thereafter, it may be collapsed for deployment as shown in Figs. 3 and 4, as will now be described.

Firstly, the stent graft assembly 10 is deformed into a straight state without collapsing the rings 12, as shown in Fig. 3. In this state, the graft 11 is deformed into the shape of a tube that is straight. This may be achieved due to the flexibility of the graft 11. As the linkages 22 are positioned on the inside of the curved shape of the graft 11, they permit this deformation to occur without stretching of the graft 11.

Fig. 3 illustrates an example in which the deformation of the graft 11 is achieved by folding of the graft 11. The folding occurs along respective pairs of fold lines 18 and 19 that are annular and extend around the graft 11. In each pair, the first fold line 18 runs along a seam 16 and the second fold line 19 runs along the piece 15 of flexible material at an angle diverging from the first fold line 18 from the inside of the curve of the graft 11 to the outside of the curve of the graft 11. The folds occur in opposite senses at the first and second fold lines 18 and 19 so that a portion of the piece 15 of flexible material between the fold lines 18 and 19 folds back over the remainder of the piece 15 of flexible material, forming a flap of material that lies along the tubular shape of the graft. Due to the divergence of the fold lines 18 and 19, this causes the relative orientation of the pieces 15 of flexible material to change until the respective longitudinal axes of the tube sections formed by the pieces 15 are aligned, and hence the graft 11 is straight. A similar type of folding may be performed when the graft 11 is moulded.

The fold lines 18 and 19 may be preformed, for example by prior folding of the graft 11 at the desired location of the fold lines 18 and 19, or by mechanical weakening of the graft 11 along the fold lines 18 and 19, for example by reducing the thickness of the graft 11 or by piercing the graft 11 along the fold lines 18 and 19.

Such folding provides the deformation mechanically, which can provide advantages. One advantage is that the folding makes the processes of deforming the graft 11 prior to delivery and deploying the graft *in situ* easy to perform, and leaves the excess material in a flap that lies tightly against the remainder of the graft 11. This facilitates the overall process of delivering the stent graft assembly 10 correctly. Another advantage is that the folding can reduce the generation of stresses within the material of the graft 11 in the deformed state, because stress are only generated on the fold lines. This can reduce the risk of damage to the stent graft assembly 10 during delivery.

As an alternative, the deformation of the graft 11 into the straight state shown in Fig. 3 may be achieved by elastic deformation of the graft 11, or by a combination of folding and elastic deformation of the graft 11.

Secondly, the stent graft assembly 10 is deformed by deforming the rings 12 into a radially collapsed state as shown in Fig. 4. As mentioned above, this is achieved by closing of the zig-zag shape of the rings 12 in the same manner as in known stent graft assemblies employing similar rings. During the radial collapse of the rings 12, the graft 11 deforms to radially collapse with the rings 12, due to being supported by the rings. 12. This radial collapse may be accommodated by the graft 11 deforming elastically or deforming by folding, or by a combination thereof. The linkages 22 do not hinder this collapse and may assist it by maintaining the separation of the rings 12. The deformation of the graft 11 is facilitated by the rings 12 being aligned with each other so that peaks of the zig-zag shape of one ring 12 are aligned with troughs of the zig-zag shape of adjacent rings 12. In the case of deformation by folding, the folds may be randomly developed creases, or may be folds along fold lines that are designed to assist the collapse. For example, such fold lines may be arranged in a pattern disclosed in WO-02/078572, US-2006/0252624 or US-2006/0265052 in respect a stent formed by a sheet of material.

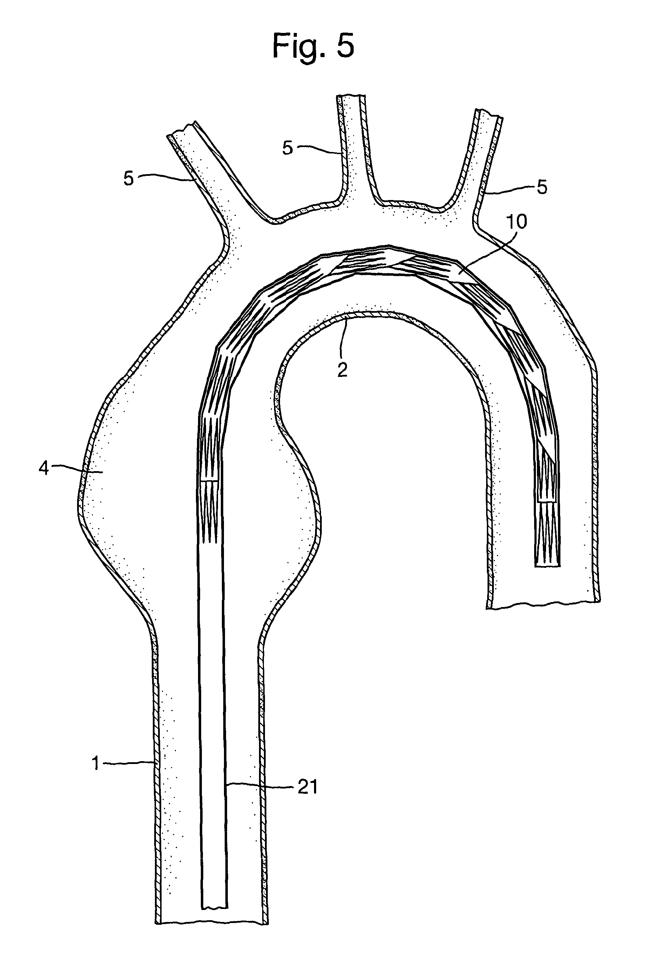

With the rings 12 of the stent frame in the radially collapsed state shown in Fig. 4, the stent graft assembly 10 is deployed in the manner shown in Figs. 5 to 7 that will now be described.

The collapsed stent graft assembly 10 shown in Fig. 4 is inserted inside a delivery catheter 20 which holds the stent frame formed by the rings 12 in a radially collapsed state. The stent graft assembly 10 is delivered to a treatment site in the arch 2 of an aorta 1 where an aneurysm 4 has formed inside the delivery catheter 20 as shown in Fig. 5. During the delivery, the stent graft assembly 10 needs to be bent to move thorough the lumens of the endovascular system, ultimately curving around the arch 2 at the treatment site.

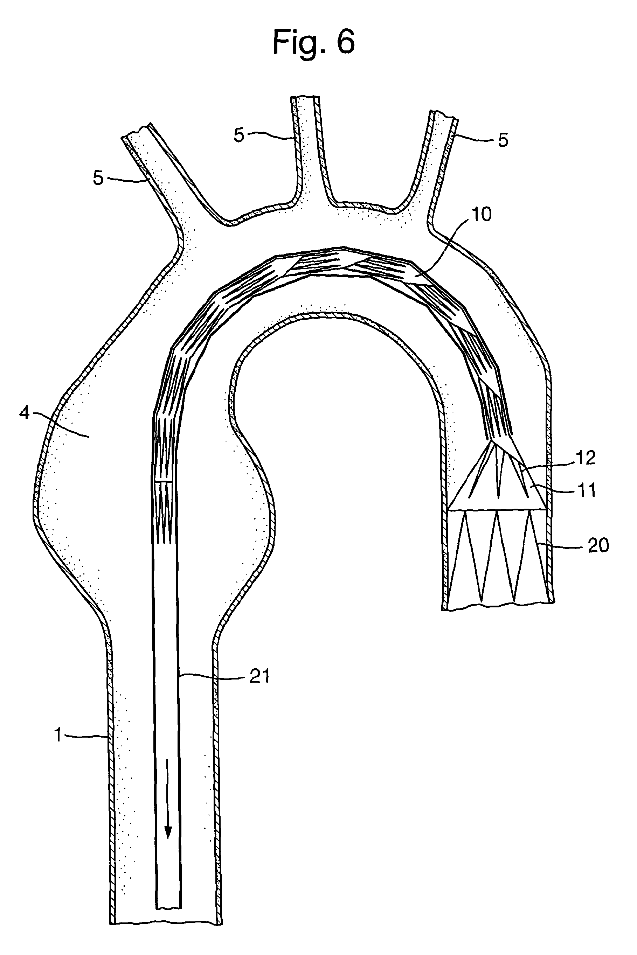

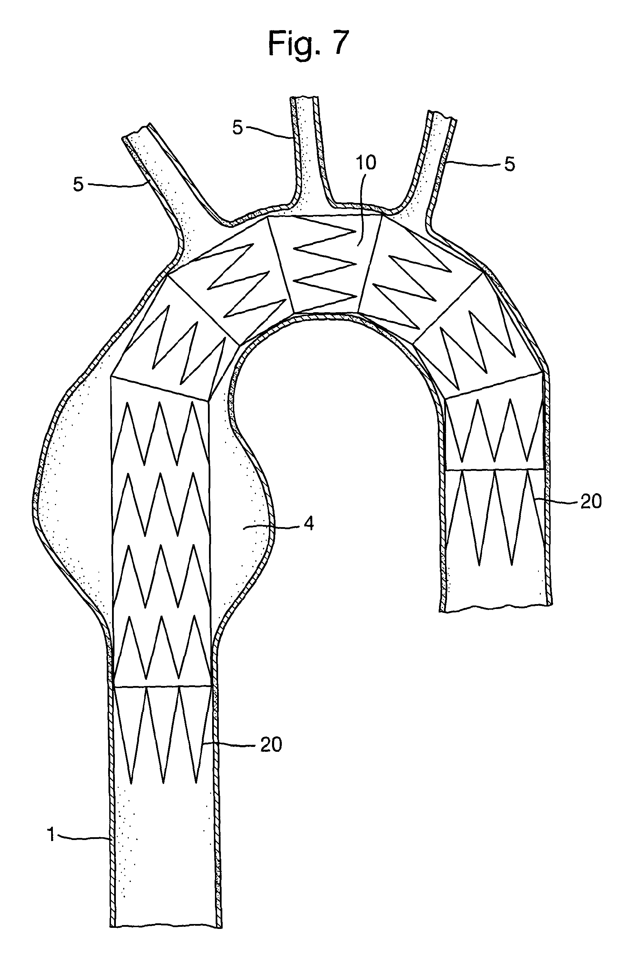

Once the stent graft assembly 10 is correctly positioned at the treatment site, the delivery catheter 21 is removed to allow the stent frame formed by the rings 12 to deform back into its expanded state. The delivery catheter 21 is first pulled slightly to release the distal end of stent graft assembly 10, as shown in Fig. 6, causing the anchor 20 to engage the aorta 1 and assist separation of the stent graft assembly 10 from the delivery catheter 21. Then, the delivery catheter 21 is further pulled back to gradually release the rest of the stent graft assembly 10, as shown in Fig. 7. During deformation into the expanded state, the stent graft assembly 10 expands into the state shown in Fig. 2 with the stent graft assembly conforming to the arch 2 of the aorta 1. The linkages 22 may assist this expansion of the graft 11.

To assist in location of the stent graft assembly 10, both in terms of its longitudinal position along the aorta 1 and in terms of its orientation around the longitudinal axis of the graft 11, the stent graft assembly 10 may be provided with radiopaque markers (not shown) that may be monitored using known techniques during the delivery.

At positions that align with the further arteries 5 when the stent graft assembly 10 is deployed at the treatment site, the stent graft assembly 10 may be provided with openings or with branches that protrude partially into the further arteries 5.

As mentioned above, the linkages 22 maintain the separation between the rings 12. During the deployment of the stent graft assembly 10 as described above, the linkages 22 prevent the rings 12 from moving together. Such movement would cause deformation of the graft 11 that is undesirable for the reasons discussed above and that would reduce the advantages of the graft 11 being curved in its expanded, undeformed state. Similarly, the linkages 22 prevent relative rotation of the rings 12 about the axis of the tubular shape during deployment. Such relative rotation of the rings 12 would introduce deformation of the graft 11 that is undesirable for the same reasons.

As the linkages 22 are arranged on the side of the graft 11 that is inside the curved shape, which is the shortest side in the curved shape, the deformation into the deformed state shown in Fig. 3 involves a reduction in the length of the other sides of the graft. Such deformation is accommodated by the folding of the graft 11 described above. Therefore the deformation avoids stretching of the graft 11 that would need to be accommodated by elastic deformation to a degree that might introduce undesirable stresses in the material of the graft 11 that risks causing damage.

Possible manufacturing techniques for the graft 11 will now be desribed.

Where the graft 11 comprises pieces 15 of flexible material joined together, the graft 11 may be made simply by cutting the pieces 15 into the desired shape and joining them together by the chosen technique. This is analogous to the tailoring of clothing such as a suit.

Where the graft 11 is moulded, the graft 11 may be manufactured by first making a mould to the desired shape and then forming a layer of material on the surface of the mould, by any suitable technique such as painting, coating or spraying. The mould may be a positive mould of the outside of the graft 11, or a negative mould of the inside of the graft 11.

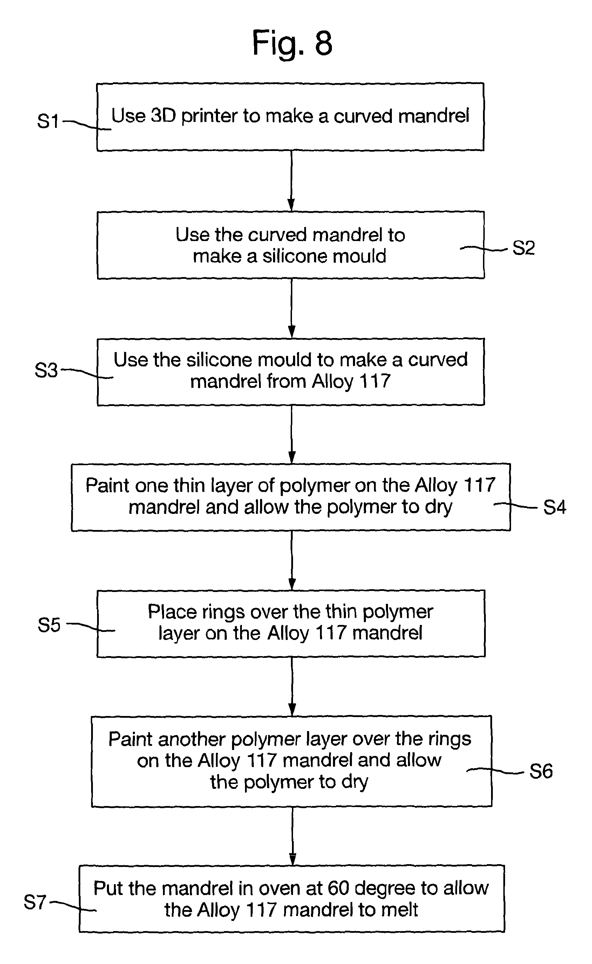

A specific example of a moulding technique is shown in Fig. 8 and will now be described in detail.

In step S1, a curved plastic mandrel is made on a 3D printer. The geometry of the mandrel is carefully defined to match the shape of TAA, which may be determined using known imaging techniques.

In step S2, the mandrel is placed in a box into which silicone mould compound (for example, SE2005 from ACC Silicone Ltd) is poured and allowed to dry before removing the plastic mandrel from the silicone mould.

In step S3, Alloy 117 is melted at an elevated temperature and poured into the silicone mould. The temperature is lowered below the melting point of Alloy 117 to form an Alloy 117 mandrel having exactly the same shape as that of the plastic mandrel. The Alloy 117 mandrel is removed from the silicone mould. Of course, a variety of alternative alloys may be used in place of Alloy 117.

In step S4, a thin layer of polymer is painted on the Alloy 117 mandrel and left to dry to form an inner layer of the graft 11.

In step S5, the rings 12 are placed over the polymer layer on the Alloy 117 mandrel.

In step S6, another thin layer of polymer is painted over the entire mandrel and left to dry to form an outer layer of the graft 11 with the rings 12 embedded therewithin.

In step S7, the entire assembly is brought to an elevated temperature higher than the melting point of Alloy 117. The Alloy 117 mandrel is allowed to melt and run away, leaving the finished stent graft assembly 10 in its expanded state.

In the above example, the stent frame is formed as a plurality of rings 12, but the stent frame could alternatively be formed by any structure that is capable of supporting the graft, allowing radial collapse and expansion, and providing longitudinal flexibility during delivery. One alternative example is to form the stent frame from rings having a slit so that the ends of the rings slide over one another on radial collapse. Another alternative example is to form the stent frame with a helical structure.

The graft 11 may be made of any suitable flexible material that is biocompatible. Non-limitative examples of possible materials are a polymer, especially but not exclusively when the graft 11 is moulded or formed by pieces 15 of material joined together, or a fabric, especially but not exclusively when the graft 11 is formed by pieces 15 joined together.

Although above the above description is directed to a stent graft assembly 10 intended to treat a TAA, the stent graft assembly 10 could be adapted for deployment at any a treatment site in a curved lumen in a human or animal body, to provide similar advantages.

## Claims

1. A stent graft assembly (10) comprising:
a graft (11) of flexible material having the shape of a tube that is curved along a longitudinal axis of the tube when the graft (11) is in an expanded, undeformed state; and
a stent frame that supports the graft (11) and is configured to be deformable between an expanded state and a radially collapsed state, the stent frame comprising a series of rings (12), each extending around the longitudinal axis of the graft (11) in a zig-zag shape,
wherein the stent graft assembly (10) is deformable into a state in which the graft (11) is deformed into the shape of a tube that is straight, and
**characterized in that** the stent graft assembly (10) further comprises linkages (22) arranged to maintain the separation between each adjacent pair of rings (12) in said series, the linkages (22) being arranged on the side of the graft (11) that is inside the curved shape when the graft (11) is in an expanded, undeformed shape.

2. A stent graft assembly (10) according to claim 1, wherein the linkages (22) are each separate elements arranged between the adjacent pairs of rings (12) in said series.

3. A stent graft assembly (10) according to any one of the preceding claims, wherein the stent graft assembly (10) is deformable into said state in which the graft (11) is deformed into the shape of a tube that is straight by folding of the graft (11).

4. A stent graft assembly (10) according to claim 3, wherein said folding occurs along one or more pairs of annular fold lines (18, 19) that extend around the stent graft assembly (10), the fold lines (18, 19) in the or each pair diverging from each other from the inside of the curve of the tube to the outside of the curve of the tube.

5. A stent graft assembly (10) according to any one of claims 1 or 2, wherein the stent graft assembly (10) is deformable into a state in which the graft (11) is deformed into the shape of a tube that is straight by elastic deformation of the graft (11).

6. A stent graft assembly (10) according to any one of the preceding claims, wherein the graft (11) is moulded to have said shape when the graft (11) is in an expanded, undeformed state.

7. A stent graft assembly (10) according to any one of claims 1 to 5, wherein the graft (11) comprises pieces (15) of flexible material joined together in a configuration providing the graft (11) with said shape of a tube that is curved when the graft (11) is in an expanded, undeformed state.

8. A stent graft assembly (10) according to claim 7, wherein said pieces (15) of flexible material joined together by stitching or bonding.

9. A stent graft assembly according to claim 7 or 8, wherein each individual piece (15) of flexible material has the shape of a tube section that is straight along a respective longitudinal axis of the tube section when the graft (11) is in an expanded, undeformed state, the pieces (15) of flexible material being joined together along seams (16) that are angled with respect to the respective longitudinal axis of the tube sections to provide the graft (11) with said shape of a tube that is curved.

10. A stent graft assembly (10) according to any one of the preceding claims, being a stent graft assembly (10) for treatment of a thoracic aorta aneurysm.

11. A method of manufacturing a stent graft assembly (10) according to any one of the preceding claims, the method comprising:
forming a graft (11) of flexible material that has the shape of a tube that is curved along a longitudinal axis of the tube when the graft is in an expanded, undeformed state and is deformable into the shape of a tube that is straight; and
supporting the graft (11) on a stent frame that is configured to be deformable between an expanded state and a radially collapsed state, the stent frame comprising a series of rings (12), each extending around the longitudinal axis of the graft (11) in a zig-zag shape, the stent graft assembly further comprising linkages (22) arranged to maintain the separation between each adjacent pair of rings (12) in said series, the linkages (22) being arranged on the side of the graft (11) that is inside the curved shape when the graft (11) is in an expanded, undeformed shape.

12. A method according to claim 11, wherein said step of supporting the graft (11) on a stent frame is performed when the stent frame is in said expanded state.

13. A method according to claim 12, wherein
said step of supporting the graft (11) on a stent frame is performed when the graft (11) is in said expanded, undeformed state, and
the method further comprises deforming the stent graft assembly (10) into a state in which the graft (11) is deformed into the shape of a tube that is straight and then deforming the stent frame into said radially collapsed state, for delivery of the stent graft assembly (10).

14. A method of manufacturing a stent graft assembly (10) according to any one of claims 11 to 13, wherein said step of forming a graft (11) of flexible material comprises moulding the graft (11) of flexible material into said shape of a tube that is curved along a longitudinal axis of the tube when the graft (11) is in an expanded, undeformed state.

15. A method of manufacturing a stent graft assembly (10) according to any one of claims 11 to 13, wherein said step of forming a graft (11) of flexible material comprises joining pieces (15) of flexible material together in a configuration providing said graft (11) of flexible material with said shape of a tube that is curved along a longitudinal axis of the tube when the graft (11) is in an expanded, undeformed state.

## Patentansprüche

1. Stentgraftanordnung (10) umfassend:
ein Graft (11) aus einem flexiblen Material, welches die Form eines Rohrs aufweist, welches entlang einer Längsachse des Rohrs gekrümmt ist, wenn das Graft (11) in einem ausgedehnten, unverformten Zustand ist; und
einen Stentrahmen, welcher das Graft (11) hält und ausgestaltet ist, zwischen einem ausgedehnten Zustand und einem radial zusammengelegten Zustand verformbar zu sein, wobei der Stentrahmen eine Reihe von Ringen (12) umfasst, welche sich jeweils um die Längsachse des Grafts (11) in einer Zickzackform erstrecken,
wobei die Stentgraftanordnung (10) in einen Zustand verformbar ist, in welchem das Graft (11) in die Form eines Rohrs verformt ist, welches gerade ist, und
**dadurch gekennzeichnet, dass** die Stentgraftanordnung (10) ferner Verbindungen (22) umfasst, welche ausgestaltet sind, die Trennung zwischen jedem benachbarten Paar von Ringen (12) in der Reihe aufrecht zu erhalten, wobei die Verbindungen (22) an der Seite des Grafts (11) angeordnet sind, welche innerhalb der gekrümmten Form ist, wenn das Graft (11) in einer ausgedehnten, unverformten Form ist.

2. Stentgraftanordnung (10) nach Anspruch 1, wobei die Verbindungen (22) jeweils getrennte Elemente sind, welche zwischen den benachbarten Paaren von Ringen (12) in der Reihe angeordnet sind.

3. Stentgraftanordnung (10) nach einem der vorhergehenden Ansprüche, wobei die Stentgraftanordnung (10) durch Falten des Grafts (11) in den Zustand verformbar ist, in welchem das Graft (11) in die Form eines Rohrs verformt ist, welches gerade ist.

4. Stentgraftanordnung (10) nach Anspruch 3, wobei das Falten entlang einem oder mehrerer Paare von ringförmigen Faltlinien (18, 19) auftritt, welche sich um die Stentgraftanordnung (10) erstrecken, wobei die Faltlinien (18, 19) in dem oder jedem Paar voneinander von der Innenseite der Krümmung zu der Außenseite der Krümmung des Rohrs divergieren.

5. Stentgraftanordnung (10) nach einem der Ansprüche 1 oder 2, wobei die Stentgraftanordnung (10) durch eine elastische Verformung des Grafts (11) in einen Zustand verformbar ist, in welchem das Graft (11) in die Form eines Rohrs verformt ist, welches gerade ist.

6. Stentgraftanordnung (10) nach einem der vorhergehenden Ansprüche, wobei das Graft (11) gestaltet ist, die Form aufzuweisen, wenn das Graft (11) in einem ausgedehnten, unverformten Zustand ist.

7. Stentgraftanordnung (10) nach einem der Ansprüche 1 bis 5, wobei das Graft (11) Teile (15) aus flexiblem Material umfasst, welche in einer Konfiguration zusammen verbunden sind, welche das Graft (11) mit der Form eines Rohrs versieht, welches gekrümmt ist, wenn das Graft (11) in einem ausgedehnten, unverformten Zustand ist.

8. Stentgraftanordnung (10) nach Anspruch 7, wobei die Teile (15) aus flexiblem Material durch Nähen oder eine Klebeverbindung miteinander verbunden sind.

9. Stentgraftanordnung nach Anspruch 7 oder 8, wobei jedes einzelne Teil (15) aus flexiblem Material die Form eines Rohrabschnitts aufweist, welcher gerade entlang einer entsprechenden Längsachse des Rohrabschnitts ist, wenn das Graft (11) in einem ausgedehnten, unverformten Zustand ist, wobei die Teile (15) aus flexiblem Material entlang Rändern (16) miteinander verbunden sind, welche winkelig bezogen auf die entsprechende Längsachse der Rohrabschnitte sind, um das Graft (11) mit der Form eines Rohrs zu versehen, welches gekrümmt ist.

10. Stentgraftanordnung (10) nach einem der vorhergehenden Ansprüche, wobei eine Stentgraftanordnung (10) für eine Behandlung eines Brustaortenaneurismas vorgesehen ist.

11. Verfahren zum Herstellen einer Stentgraftanordnung (10) nach einem der vorhergehenden Ansprüche, wobei das Verfahren umfasst:
Ausbilden eines Grafts (11) aus einem flexiblen Material, welches die Form eines Rohrs aufweist, welches entlang einer Längsachse des Rohrs gekrümmt ist, wenn das Graft in einem ausgedehnten, unverformten Zustand ist, und in die Form eines Rohrs verformbar ist, welches gerade ist; und
Halten des Grafts (11) an einem Stentrahmen, welcher ausgestaltet ist, zwischen einem ausgedehnten Zustand und einem radial zusammengelegten Zustand verformbar zu sein, wobei der Stentrahmen eine Reihe von Ringen (12) umfasst, welche sich jeweils um die Längsachse des Grafts (11) in einer Zickzackform erstrecken, wobei die Stentgraftanordnung ferner Verbindungen (22) umfasst, welche angeordnet sind, um die Trennung zwischen jedem benachbarten Paar von Ringen (12) in der Reihe aufrecht zu erhalten, wobei die Verbindungen (22) an der Seite des Grafts (11) angeordnet sind, welche innerhalb der gekrümmten Form ist, wenn das Graft (11) in einem ausgedehnten, unverformten Zustand ist.

12. Verfahren nach Anspruch 11, wobei der Schritt des Haltens des Grafts (11) an einem Stentrahmen ausgeführt wird, wenn der Stentrahmen in dem ausgedehnten Zustand ist.

13. Verfahren nach Anspruch 12, wobei
der Schritt des Haltens des Grafts (11) an einem Stentrahmen ausgeführt wird, wenn das Graft (11) in dem ausgedehnten, unverformten Zustand ist, und
das Verfahren ferner ein Verformen der Stentgraftanordnung (10) in einen Zustand, in welchem das Graft (11) in die Form eines Rohrs verformt ist, welches gerade ist, und dann ein Verformen des Stentrahmens in den radial zusammengelegten Zustand umfasst, um die Stentgraftanordnung (10) einzuführen.

14. Verfahren zum Herstellen einer Stentgraftanordnung (10) nach einem der Ansprüche 11-13, wobei der Schritt des Formens eines Grafts (11) aus flexiblem Material ein Gestalten des Grafts (11) aus flexiblem Material in die Form eines Rohrs umfasst, welches entlang einer Längsachse des Rohrs gekrümmt ist, wenn das Graft (11) in einem ausgedehnten, unverformten Zustand ist.

15. Verfahren zum Herstellen einer Stentgraftanordnung (10) nach einem der Ansprüche 11-13, wobei der Schritt des Formens eines Grafts (11) aus flexiblem Material ein Zusammenverbinden von Teilen (15) aus flexiblem Material in einer Konfiguration umfasst, welche das Graft (11) aus flexiblem Material mit der Form eines Rohrs versieht, welches entlang einer Längsachse des Rohrs gekrümmt ist, wenn das Graft (11) in einem ausgedehnten, unverformten Zustand ist.

## Revendications

1. Système (10) pour greffe d'endoprothèse, comportant :
une greffe (11) en matière souple ayant la forme d'un tube incurvé suivant un axe longitudinal du tube quand la greffe (11) est en configuration déployée, sans déformation ; et
une armature d'endoprothèse qui supporte la greffe (11) et est conçue pour pouvoir se déformer entre une configuration déployée et une configuration radialement comprimée, l'armature d'endoprothèse comprenant une série de bagues (12) s'étendant chacune en zigzag autour de l'axe longitudinal de la greffe (11),
le système (10) pour greffe d'endoprothèse pouvant se déformer pour prendre une configuration dans laquelle la greffe (11) se transforme en un tube rectiligne, et
le système (10) pour greffe d'endoprothèse étant **caractérisé en ce qu'**il comporte en outre des liaisons (22) conçues pour maintenir la séparation entre chaque paire adjacente de bagues (12) de ladite série, les liaisons (22) étant installées du côté de la greffe (11) situé à l'intérieur de la forme incurvée quand la greffe (11) est en configuration déployée, sans déformation.

2. Système (10) pour greffe d'endoprothèse selon la revendication 1, dans lequel les liaisons (22) sont, chacune, des éléments séparés disposés entre les paires adjacentes de bagues (12) de ladite série.

3. Système (10) pour greffe d'endoprothèse selon l'une quelconque des revendications précédentes, le système (10) pour greffe d'endoprothèse étant déformable pour prendre ladite configuration dans laquelle la greffe (11) se transforme, par pliage de la greffe (11), en un tube rectiligne.

4. Système (10) pour greffe d'endoprothèse selon la revendication 3, dans lequel ledit pliage a lieu suivant une ou plusieurs paires de lignes de pliage annulaires (18, 19) qui s'étendent autour du système (10) pour greffe d'endoprothèse, les lignes de pliage (18, 19) de la/de chaque paire divergeant les unes des autres depuis l'intérieur de la courbure du tube vers l'extérieur de la courbure du tube.

5. Système (10) pour greffe d'endoprothèse selon l'une quelconque des revendications 1 et 2, dans lequel le système (10) pour greffe d'endoprothèse est déformable pour prendre une configuration dans laquelle la greffe (11) se déforme pour se transformer, par déformation élastique de la greffe (11), en un tube rectiligne.

6. Système (10) pour greffe d'endoprothèse selon l'une quelconque des revendications précédentes, dans lequel la greffe (11) est moulée pour avoir ladite forme quand la greffe (11) est dans une configuration déployée, non déformée.

7. Système (10) pour greffe d'endoprothèse selon l'une quelconque des revendications 1 à 5, dans lequel la greffe (11) comprend des morceaux (15) de matière souple réunis les uns aux autres dans un agencement constituant la greffe (11) avec ladite forme d'un tube incurvé quand la greffe (11) est dans une configuration déployée, non déformée.

8. Système (10) pour greffe d'endoprothèse selon la revendication 7, dans lequel lesdits morceaux (15) de matière souple sont réunis les uns aux autres par des points de couture ou par collage.

9. Système (10) pour greffe d'endoprothèse selon la revendication 7 ou 8, dans lequel chaque morceau individuel (15) de matière souple a la forme d'un segment de tube qui est rectiligne sur un axe longitudinal respectif du segment de tube quand la greffe (11) est dans une configuration déployée, non déformée, les morceaux (15) de matière souple étant réunis les uns aux autres le long de jonctions (16) qui sont obliques par rapport à l'axe longitudinal respectif des segments de tube afin de réaliser la greffe (11) avec ladite forme de tube rectiligne.

10. Système (10) pour greffe d'endoprothèse selon l'une quelconque des revendications précédentes, étant un système (10) pour greffe d'endoprothèse pour le traitement d'un anévrisme de l'aorte thoracique.

11. Procédé de fabrication d'un système (10) pour greffe d'endoprothèse selon l'une quelconque des revendications précédentes, le procédé comportant :
la formation d'une greffe (11) en matière souple ayant la forme d'un tube incurvé suivant un axe longitudinal du tube quand la greffe est en configuration déployée, sans déformation, et déformable pour se transformer en tube rectiligne ; et
le soutien de la greffe (11) par une armature d'endoprothèse conçue pour pouvoir se déformer entre une configuration déployée et une configuration radialement comprimée, l'armature d'endoprothèse comprenant une série de bagues (12) s'étendant chacune en zigzag autour de l'axe longitudinal de la greffe (11), le système comportant en outre des liaisons (22) conçues pour maintenir la séparation entre chaque paire adjacente de bagues (12) de ladite série, les liaisons (22) étant installées du côté de la greffe (11) situé à l'intérieur de la forme incurvée quand la greffe (11) est en configuration déployée, sans déformation.

12. Procédé selon la revendication 11, dans lequel ladite étape de soutien de la greffe (11) sur une armature d'endoprothèse est effectuée quand l'armature d'endoprothèse est dans ladite configuration déployée.

13. Procédé selon la revendication lé, dans lequel
ladite étape de soutien de la greffe (11) sur une armature d'endoprothèse est exécutée quand la greffe (11) est dans ladite configuration déployée, sans déformation, et
le procédé comporte en outre une déformation du système (10) pour greffe d'endoprothèse pour prendre une configuration dans laquelle la greffe (11) se transforme en un tube rectiligne, puis une déformation de l'armature d'endoprothèse pour qu'elle prenne ladite configuration radialement écrasée, pour l'obtention du système (10) pour greffe d'endoprothèse.

14. Procédé de fabrication d'un système (10) pour greffe d'endoprothèse selon l'une quelconque des revendications 11 à 13, dans lequel ladite étape de formation d'une greffe (11) en matière souple comprend un moulage de la greffe (11) en matière souple pour lui donner ladite forme d'un tube incurvé suivant un axe longitudinal du tube quand la greffe (11) est dans une configuration déployée, non déformée.

15. Procédé de fabrication d'un système (10) pour greffe d'endoprothèse selon l'une quelconque des revendications 11 à 13, dans lequel ladite étape de formation d'une greffe (11) en matière souple comprend la réunion de morceaux (15) de matière souple les uns avec les autres dans un agencement constituant la greffe (11) en matière souple avec ladite forme d'un tube incurvé suivant un axe longitudinal quand la greffe (11) est dans une configuration déployée, non déformée.
